Europäisches Patentamt

European Patent Office

Office européen des brevets

(19)

(11) Publication number: **0 166 009**
**A1**

## EUROPEAN PATENT APPLICATION

(12)

(21) Application number: **84107205.1**

(22) Date of filing: **22.06.84**

(51) Int. Cl.⁴: **A 61 K 6/08**

(43) Date of publication of application: **02.01.86**
**Bulletin 86/1**

(84) Designated Contracting States: **AT BE CH DE FR GB IT LI LU NL SE**

(71) Applicant: **DENTSPLY GMBH, Eisenbahnstrasse 180 Postfach 201 160, D-6072 Dreieich (DE)**

(72) Inventor: **Goerlich, Karl Joachim, Dr., Forststrasse 31, D-6149 Fuerth/Odenwald (DE)**

(74) Representative: **Wächtershäuser, Günter, Dr., Tal 29, D-8000 München 2 (DE)**

(54) **Photocurable dental composition for crown and bridge purposes.**

(57) A photopolymerizable dental composition for preparing crowns and bridges is described. It contains a photo initiator system as well inorganic and/or organic filler additionally to polyfunctional (meth)acrylate. The polyfunctional acrylate comprises a reaction product of a polyisocyanate and a di glycidyl (meth)acrylate of a bisphenol or oligomers thereof as well as liquid polyfunctional (meth)acrylate suitable for dissolving said reaction product.

EP 0 166 009 A1

EAD-4327

DENTSPLY GMBH
Dreieich, West Germany

---

Photocurable Dental Composition for Crown
and Bridge Purposes

---

The present invention relates to improved dental compositions
and particularly to photocurable dental compositions for crown
and bridge purposes.

In the long standing rivalry between ceramic and polymerizable compositions for preparing crowns and bridgework, the
latter have received increasing preference mostly because
of reduced costs, easy processing and increased impact
strength. For a long time the only practically feasible
polymerization system was a heat polymerizable two-component
system, one component containing the polymerizable monomers
and the other component containing the peroxide initiator.
While such systems lead to polymerized end products with
satisfactory properties, they must be marketed in the two
component form, since a one component composition would have
an intolerably short shelf life. Two-component systems, however,
are inflicted with numerous handling and processing disadvantages. Frequently the two components are not acurately
measured, leading to less than optimum compositions. Further
inhomogeneous products result from incomplete mixing or from
the introduction of air bubbles during mixing. After mixing, the pot life is rather short, requiring rapid and skillful manipulation in building up the desired dental work, layer after layer. This difficulty leads often to the waste of material which has gone beyond the tolerable pot life. Finally the high temperature polymerization leads to shrinkage and undesirable gaps. If more reactive radical starters are used in a cold polymerization process, the pot life is intolerably short.

For these reasons photopolymerizable one-component compositions for crowns and bridges have been developped, which have a long shelf life. However, these compositions are inflicted with a dilemma. A sufficiently high impact strength of the end product is obtained only with compositions which have a rather low viscosity and which therefore require a large number of intermediate irradiations in the manufacturing of the crowns and bridges. On the other hand prior art compositions with a pasty consistency lead to an insufficient impact strength.

Further, it has sometimes been observed that an increase in irradiation time leads to a decrease of the impact strength. On the other hand a sufficiently long and extensive irradiation is necessary in order to bring the polymerization process to completion. Otherwise, slow dark reactions will occur over a period of several days, accompanied by an equally slow change of color, usually toward lighter color tones. This makes the adjustment of precise color tones quite difficult.

It is therefore a first object of the present invention to prepare a photopolymerizable dental composition for crown and bridge purposes which has a pasty consistency and which leads to products with a high impact strength.

- 3 -

It is a further object of the present invention to provide
a photopolymerizable dental composition for crown and
bridge purposes which can be processed with relatively short
irradiation times and which leads to products with high
color stability. These and other objects are achieved
with a photopolymerizable dental composition for crown
and bridge purposes with a content of a polyfunctional
(meth)acrylate; and organic and/or inorganic filler and
a photoinitiator system, which is characterized in that
the polyfunctional (meth)acrylate comprises a reaction
product of a polyisocyanate and a diglycidyl (meth)acrylate
of a bisphenol or oligomers thereof and a liquid polyfunctional (meth)acrylate for dissolving said reaction product.

It has been found that the content of a reaction product
of a polyisocyanate and a bis-glycidyl (meth)acrylate
of a bisphenol leads to high impact strength values even
if the composition is adjusted for a pasty consistency.
Further, the compositions may be cured with visible light
in a relatively short period of time. A stable color
tone is quickly reached and the product does not suffer
slow color changes after the termination of the irradiation.
Notably, the end products are color-stable in water and in
the mouth. Finally, the end products have a high abrasion
resistence and they exhibit superior polishing characteristics.

It is preferred to use as an inorganic filler a combination
of pyrogenic silica and glass for an increased impact strength,
bending strength and abrasion resistence.

- 4 -

The photopolymerizable composition of the present invention contains a system of polyfunctional (meth)-acrylates for establishing the polymer matrix. It comprises as a first essential component a reaction product of a polyisocyanate, preferably a diisocyanate and a diglycidyl (meth)acrylate of a bisphenol, preferably bisphenol A. Such reaction products are described in detail in US-patent 3,629,187 and reference is made explicitly to this publication for disclosure purposes. Preferably the two reaction components are used in about a 1:1 molar ratio. In the following description this reaction product will be referred to briefly as NCO-monomer.

The liquid polyfunctional (meth)acrylate component may consist of one or several such polyfunctional (meth)acrylates. It is preferred to combine a dimethacrylate with a tri-(meth)acrylate. The di(meth)acrylate may be derived from ethylene glycol, diethylene glycol, triethylene glycol, tetraethylene glycol, polyglycol-400, 1,3-propanediol, 1,4-butanediol, 1,6-hexanediol, 1,10-dodecanediol, 1,12-dodecanediol, 1,4-cyclohexanediol, 1,3-pentanediol, neopentylglycol, glycerol, pentaerythritol etc. Further, various dimethacrylates of bisphenol A derivatives may be used. The latter may be formally derived as a reaction product of bisphenol A and one or more molecules (on either side) of ethylene oxide, propylene oxide, further a reaction product of hydroxyalkyl (meth)acrylate with a diisocyanate such as trimethylhexamethylene diisocyanate or isophoronediisocyanate may be used.

The tri(meth)acrylate may be derived from trimethylolpropane or from pentaerythritol, glycerol or 1,2,4-butanetriol, trimethylolethane, trimethylolmethane. Further, a tetra(meth)acrylate of a tetraol such as tetramethylolmethane or pentaerithritol may be used.

- 5 -

The weight ratio of the NCO-monomer to the liquid polyfunctional
(meth)acrylate should be chosen so that the NCO-monomer dissolves.
Preferably, the weight ratio is in the range of from
0.05 : 1 to 5 : 1 and especially in the range of from
0.1 :1 to 2 : 1 and more especially in the range of from
0.2 :1 to 1.5:1. Tri, tetra and higher liquid (meth)acrylates
may be absent. If they are present the weight ratio of the
liquid di(meth)acrylate to the liquid tri and higher (meth)acrylate
should be preferably  above    0.1 : 1 and preferably 20 : 1
to 1 : 1 and more preferably 15 : 1 to 2 : 1.


Next the photo initiator system shall be described. Usually
                                                  a ketone and/or
the photo initiator is a combination of/ an α-diketone and
a reducing agent. It is most preferable to use camphoroquinone,since it provides the best results in cooperative
relation to the NCO-monomer. But other   diketones may also
be used such as biacetyl, benzyl or derivatives thereof,
benzoin or derivatives thereof, fluorenone, α- or β-naphthil
or the like.


Various types of reducing agents may be used.  It is highly preferable to use a tertiary amine or its salt as reducing agent such as
N,N-diethanolmethylamine or triethanolamine or (meth)acrylates
thereof. 2-Dimethylaminoethyl(meth)acrylate and 3-dimethyl-
aminoneopentyl(meth)acrylate are preferred. It is most preferable
to use a combination of such an amine and (meth)acrylic
acid (preferable at a molar ratio of about 1 : 1), i.e.
a salt of said amine with said acid. The latter two amines
and particularly their (meth)acrylic acid salts are particularly preferred since they exhibit a cooperative effect with
camphoroquinone and theNCO-monomer in terms of improved
discoloration of the sensitizer and in terms of improved
color stability of the product. It is however also possible
to use other reducing agents such as the barbituric acid
derivatives as described in German patent application
31 36 484 , or sulfinic acids, sulfinates or the like.
The preparation of organic amine salts is described  in
European Patent Application 84 102 448.2 The content of the ketone and/or diketone, based on the total composition is preferably within the range of from 0.0005 to 2 weight-% and more preferably 0.001 to 0.8 weight-%. The content of the reducing agent or amine or amine salt is preferably in the range of from 0.01 to 5 weight-% and more preferably in the range of 0.03 to 3 weight-% based on the total composition.

The composition of the present invention may contain one or several inorganic fillers and/or one or several organic fillers. The weight ratio of the total amount of fillers to the total amount of monomers should be in the range of preferably 95 : 5 to 5 : 95 and especially 90 : 10 to 10 : 90. Most preferably the weight ratio of the fillers to the monomers is in the range of 1 : (0.4 to 1) and especially 1 : (0.5 to 0.9).

The filler composition may include (a) an inorganic filler or (b) an organic filler prepared from prepolymerized resin, which itself may contain an inorganic filler, or (c) a combination thereof. The inorganic filler may be silaned pyrogenic or precipitated silica and additionally alumina, titanium oxide, magnesium oxide, inorganic glass, quartz, sodium-alumina-silicate or any other customary inorganic filler.

Preferably the filler composition contains a considerable amount of pyrogenic silica. It is most preferable to use a highly disperse silanized pyrogenic silica as sold by Wacker (HDK). This may have a variety of different BET surfaces and densities. It is particulary preferred to use silica with a BET surface of 170 $m^2$/g and a bulk density of 90 g/l. It is also possible to use an Aerosil (Degussa). The BET surface is preferably in the range of from 10 to 400 and especially in the range of from 50 to 300 $m^2$/g. The filler composition should contain a total amount of silane-treated pyrogenic silica of preferably 10 to 100 weight-% and especially 20 to 90 weight-% and most particularly of 30 to 80 weight-%. Of course different types of silane-treated pyrogenic silica with different BET-surface may be combined.

Further, the filler composition may contain an organic filler. The filler composition may contain 0 to 100 % of the organic filler and preferably 10 to 90 weight-% of the organic filler and especially 30 to 85 weight-%. The organic filler may be a cross-linked polymer with or without a content of an inorganic filler. The organic filler without a content of an inorganic filler may be a polymethyl(meth)acrylate, which is cross-linked with one of the di- or poly(meth)acrylates mentioned above. Various other conventional types of such organic fillers may be also used. These organic fillers may be prepared with radical-forming initiators under hot or cold conditions, whereby for example peroxides may be used or by photopolymerization under the usual conditions, for example with the above-mentioned initiators. Preferably the organic filler contains an inorganic filler as mentioned above and notably a silane-treated pyrogenic or precipitated silica, as mentioned above. The content of such inorganic filler should be in the range of from 10 to 90 weight-% and preferably 20 to 80 weight-% and especially 40 to 80 weight-%. It is particulary preferred to use an organic filler which has been prepared from a composition with a content of an NCO-monomer and one of the other liquid polyfunctional (meth)acrylates as mentioned above. As to the possible and preferred types and ratios of the monomers reference is made to the explanations given above for the dental composition. Again these organic fillers may either be produced by using a peroxide catalyst or by photopolymerization under the above-mentioned conditions. The organic filler may be a bead polymer or a fractured polymer.

It should be noted, that there is a special cooperative effect between the NCO-monomer and the pyrogenic silica, mentioned above and this cooperative or synergistic effect in terms of the desirable mechanical characteristics holds for the organic filler as well as for the dental composition and it is preferred to utilize this effect both with respect to the monomers and inorganic filler of the dental composition and the monomers and inorganic filler in the organic filler of the dental composition.

Preferably, the dental composition contains glass as an additional inorganic filler. This glass may be partly or completely contained within the organic filler or the organic filler may be free of a glass content. In any event the amount of glass to the total amount of inorganic filler including the inorganic filler within the organic filler is in the range of up to 60 wt.-%, preferably 26 to 40 wt.-% and most preferably 30 to 40 wt-%, based on the total amount of inorganic filler. The glass should have a refractory index close to that of the resin matrix for proper light transmission. It is of particular advantage, that the photocurable dental composition may be given a pasty consistency. This consistency may be adjusted by the ratio of liquid monomers to the total amount of the inorganic filler, the organic filler and the NCO-monomer.

Further, the dental composition may contain various usual additives, for example inhibitors, for example p-methoxyphenol and UV-stabilizors, fluorescent materials, plasticizers, pigments and so forth.

The dental composition may be used for preparing crowns, bridges and the like in the usual manner. It will not stick to the spatula and it may have a consistency which is most suitable for the required manipulations. The photopolymerization may be conducted in stages either for each layer or for several layers at a time or once for all layers. A curing depth of at least 2 mm is reached within 5 minutes. Visible light in the range of 350 to 700 nm especially 400-600 nm is preferred.

The following non-limiting examples further illustrate certain preferred embodiments of the invention. If not specified otherwise, $\gamma$-methacrylqxypropyl-trimethoxy-silane was used for silaning the inorganic fillers. The term "NCO-monomer" whenever used in the examples refers actually to a mixture of 1 part by weight of triethyleneglycoldimethacrylate and 1 part by weight of a reaction product of 1 mole hexamethylene diisocyanate and 1 mole of a diester of bisphenol A with glycidylmethacrylate.

## Example 1

In a mixing vessel 30 g 1,4-butanedioldimethacrylate (BDDMA)
and 10 g trimethylolpropanetrimethacrylate (TMPTMA) were
mixed, whereafter 0.7 g DL-camphoroquinone, 2.0 g dimethylaminoethylmethacrylate (DMAEMA) and 1.2 g methacrylic acid
(MAA) were dissolved under stirring. Further, the desired
amount of fluorescent material, hydroquinone and so forth
as well as the necessary pigments . were added. ·
Next 120 g of NCO-monomer were added, which had been
previously prepared by reacting equimolar amounts of
hexamethylenediisocyanate and bisphenol-A-glycidyl-methacrylate.
Further 100 g of a silane-treated pyrogenic silica with a
specific surface (BET) of about 170 $m^2$/g and a bulk density
of about 90 g/l (HDK H 2000, produced by Wacker) were added.
Finally, 100 g of an organic filler with a particle size of
less than 100 micron were added. The organic filler had been
prepared by polymerizing 99.2 weight-% methylmethacrylate
and 0.8 % ethyleneglycoldimethacrylate without an inorganic
filler in the usual manner.

This composition was thoroughly mixed. A pasty composition was
obtained. It was used for preparing crowns and bridges by a standard
build-up technique, followed by irradiation with visible light for
7 minutes using a curing unit delivering more than 50 mW/$cm^2$ between
400 and 500 nm. Test samples were prepared in the same manner and
evaluated according to DIN 53435. An impact strength of
1.2 kJ/$m^2$ was measured. The product was free of any yellow color
due to the camphoroquinone and it did not suffer color changes,
when tested according to DIN 13907.

## Comparative example 1

E xample 1 was repeated with the exception that instead of
the NCO-monomer 120 g of a product obtained by reacting
hydroxyethylmethacrylate and trimethylhexamethylenediiso- cyanate in a molar ratio of 2 : 1 (Plex 6661,Roehm) were
used. After an irradiation of 7 minutes as before the impact
strength was not higher than 0.4 kJ/m$^2$, when tested according to
DIN 53435.

Examples 2 and 3

Example 1was repeated with the exception that BDDMA was replaced
by tetraethyleneglycoldimethacrylate (TTEG) or hexanediol-
dimethacrylate (HD) with similarly excellent results in terms
of the impact strength, other mechanical properties and
color stability.

Example 4

Example 1was repeated, whereby 80 g of the NCO-monomer, 70 g
HD and 10 g TMPTMAwere used as monomers together with 0.5 g
pigments, 0.2 g camphoroquinone, 0.34 g methacrylic acid and
0.57 g DMAEMA. As inorganic filler 17 g HDKH 2000 was used
together with 80 g of silaned Aerosil OX 50 (Degussa) with
a BET surface of 50 m$^2$/g and a bulk density of about 80 g/l
and a primary particle size of about 40 nm. Further, 100 g
of an organic fillerwere used.

The organic fillerwas prepared by photopolymerization of
a mixture of 0.13 % camphoroquinone 0.22 % methacrylic acid,
0.36 % DMAEMA, 27.33 % Plex 6661-0, 3.64 % BDDMA and
86.32 % of an Aerosil mixture consisting of 98 % silane-
treated Aerosil OX 50 and 2 % Aerosil R 972. This polymer
wasground to a particle size of less than 65 micron.

Again excellent results in terms of impact strength, other
mechanical properties and color quality and color stability
were achieved.

Example 5

Example 4 wasrepeated except that HDwas replaced by 70 g
of 1,12-dodecanediol dimethacrylate (DD). Further, 0.1 g
camphoroquinonewascombined with 0.3 g of the methacrylic acid salt of 3-dimethylamino neopentyl acrylate (SH), prepared
according to example 1 of European Patent Application 84 102 448.2
Further, instead of the organic filler used in example 4
a different organic filler was used which was prepared by
photopolymerization of a composition consisting of 0.06
weight-% camphoroquinone, 0.12 weight-% SH, 21.73 weight-%
DD, 0.68 weight-% BDDMA and 77.41 weight-% of a mixture
of silane-treated Aerosil OX 50 (99 weight %) and Aerosil
R 972 (1 weight-%).

Again excellent mechanical qualities including a high impact
strength were combined with a good color stability and an
excellent color quality.

Example 6

Example 5 was repeated, with the exception that 270 g of the
organic filler of example 5 were used as the sole filler
material, while 40 g of DD and 50 g of the NCO-monomer were
used as the sole monomers. Again excellent mechanical properties including a high impact strength and excellent color
stability and color qualities were obtained.

Example 7

Example 5 was repeated, whereby 35 g HD, 40 g NCO-monomer and
5 g TMPTMA were used as monomer mixture, combined with 0.3 g
pigments, 0.15 g of camphoroquinone and 0.6 g SH. A filler
composition consisting of three fillers was used, namely 55.0 g
of HDK H 2000 and 20 g of the Aerosil filler of example 5
and 50 g of an organic filler.

The organic filler was prepared by photopolymerization of a
composition of 0.056 % camphoroquinone, 0.111 % SH, 0.997 TMPTMA
and 29.95 % NCO-monomer, combined with 67.887 weight-% of
an Aerosil filler consisting of 99 weight-% silane-treated

Aerosil OX 50 and 1 weight-% Aerosil R 972. Again an excellent dental composition with satisfactory mechanical and optical properties was obtained.

Example 8

Example 1 was repeated with 150 g of the NCO-monomer, 10 g triethyleneglycoldimethacrylate, 90 g HDK H 2000, silaned with 5 g $\gamma$-methacryloxypropyl-trimethoxy-silane, 60 g silaned glass (Schott glass P 8412, silaned with $\gamma$-methacryloxypropyl-trimethoxy-silane), 0.13g camphoroquinone, 0.63 g SH and 100 g of an organic filler containing the required pigment. (The organic filler was obtained by fracturing a polymer composition prepared from 0,056% camphoroquinone, o,291% SH, 29.950% NCO monomer, 1,977% Trimethylol-propanetrimethacrylate and 67,706% of a mixture of 99% Aerosil 0X50 (silaned) and 1% Aerosil R 972). A pasty composition was obtained. The photocured end product had an impact strength of 2,3 kJ/m$^2$, when tested according to DIN 53435.

Example 9

Example 8 was repeated, whereby 28 % of the inorganic filler within the organic filler was substituted by silaned glass (Schott glass P 8412), particle size 100% $<$10µm, 50% 10 - 4µm and 50% $<$4µm. Again a pasty composition was obtained, which led to an excellent impact strength.

Example 10

Example 1 was repeated whereby a previously prepared salt of 2.0 g DMAEMA and 1.2 g MAA(obtained in accordance with example 2 of European Patent Application 84 102 448.2) was used. The composition showed a high polymerization rate. The end product had a very high impact strength.

Patent Claims

1.     A photopolymerizable dental composition for preparing crowns and bridges with a content of
(a) polyfunctional (meth)acrylate,
(b) organic and/or inorganic filler,
(c)     an initiator system,
characterized in that the polyfunctional (meth)acrylate comprises a reaction product of a polyisocyanate and a diglycidyl (meth)acrylate of a bisphenol or oligomers thereof and a liquid polyfunctional (meth)acrylate for dissolving said reaction product.

2.     A composition according to Claim 1, characterized in that the initiator is a photo initiator, preferably a combination of a ketone and/or an $\alpha,\beta$-diketone and an amine or an amine salt.

3.     A composition according to Claim 2, characterized in that the amine component is dimethylaminoethyl (meth)acrylate or a (meth)acrylic acid salt thereof; or dimethylamino-neopentyl(meth)acrylate or a (meth)acrylic acid salt thereof or triethanol amine or a (meth)acrylic acid salt thereof.

4.     A composition according to one of Claims 1 to 3, characterized by a content of a silane-treated pyrogenic silica as inorganic filler.

5.     A composition according to one of Claims 1 to 4, characterized in that the organic filler has been obtained from a composition comprising a silane-treated pyrogenic silica and a reaction product of a diisocyanate and a diglycidyl (meth)acrylate of a bisphenol or oligomers thereof.

6.    A composition according to one of the Claims 1 to 5, characterized in that the inorganic filler in said dental composition comprises glass, which is contained inside and/or outside said organic filler.

7.    A composition according to one of the Claims 2 to 6, characterized in that said photoinitiator is a combination of camphoroquinone and an amine.

8.    A composition according to one of Claims 2 to 6, characterized in that said photoinitiator is a combination of a diketone and an amine salt.

9.    A composition according to one of Claims 1 to 8, characterized by a combination of an inorganic filler comprising silane-treated pyrogenic silica and an organic filler, comprising silane-treated pyrogenic silica dispersed within the crosslinked polymer matrix.

10.    A composition according to one of Claims 1 to 8, characterized by a combination of an inorganic filler, comprising silane-treated pyrogenic silica and silane-treated glass and an organic filler, containing silane-treated pyrogenic  silica and optionally silane-treated glass dispersed within the crosslinked polymer matrix.

11.     A composition according to one of claims 1 to 10, characterized by

(a) 5 to 95 parts by weight of a polyfunctional (meth)acrylate comprising

    (aa) 0.05 to 5 parts by weight of the reaction product of the polyisocyanate and the diglycidyl(meth)acrylate of a bisphenol or oligomers thereof and

    (ab) 1 part by weight of a liquid polyfunctional (meth)acrylate for dissolving said reaction product (aa);

(b) 95 : 5 parts by weight of a filler composition comprising

    (ba) 10 to 90 weight % of an inorganic filler comprising silaned pyrogenic silica and silaned glass and

    (bb) 90 to 10 weight % of a  cross-linked organic filler comprising

        (bba) 90 to 10 weight % of a polymer matrix obtained by copolymerization of the polyfunctional(meth)-acrylates (a) and/or other solid and liquid (meth)acrylates, preferably with the photoinitiator system (c) and

        (bbb) 10 to 90 weight % of an inorganic filler comprising silaned pyrogenic silica and optionally silaned glass whereby the filler composition contains a total amount of 20 to 90 weight % of silaned pyrogenic silica and whereby the total amount of silaned glass is 10 to 60 weight % based on the total amount of inorganic filler and

(c) a photoinitiator system of a ketone or α-diketone and an amine or an amine salt.

European Patent
Office

**EUROPEAN SEARCH REPORT**

Application number

EP 84 10 7205

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,Y | DE-A-2 126 419 (DENTSPLY INT. INC.) <br> * Page 1; page 2, paragraph 2; page 3, paragraph 1; page 4, paragraph 2; pages 10-17; example 3; claims * | 1-11 | A 61 K 6/08 |
| X,Y | DE-A-2 226 154 (BAYER) <br> * Page 2, lines 28-34; claims * | 1-11 | |
| X,Y | FR-A-2 285 118 (LOCTITE CORP.) <br> * Page 1, paragraphs 1,2; page 3, line 11 - page 8, line 37; claims * | 1-11 | |
| Y | US-A-4 411 625 (F.F. KOBLITZ) <br> * Column 4, lines 34-54; column 5, line 19 - column 6, line 56; claims 1-3 * | 1-11 | TECHNICAL FIELDS SEARCHED (Int. Cl.4) |
| D,X <br> Y | US-A-3 629 187 (D.E. WALLER) <br><br> * Whole document * | 1-11 | A 61 K 6/00 |
| X,Y | FR-A-2 271 807 (I.C.I.) <br> * Page 4, line 10 - page 5, line 11; claims 1-5,9-10 * | 1-11 | |
| X,Y | DE-A-3 133 008 (DENTSPLY INT. INC.) <br> * Claims * | 1-11 | |

--- -/-

The present search report has been drawn up for all claims

| Place of search <br> THE HAGUE | Date of completion of the search <br> 12-12-1984 | Examiner <br> BERTE M.J. |
|---|---|---|

0166009

European Patent
Office

**EUROPEAN SEARCH REPORT**

Application number

EP 84 10 7205

| | DOCUMENTS CONSIDERED TO BE RELEVANT | | Page 2 |

| Category | Citation of document with indication, where appropriate, of relevant passages | Relevant to claim | CLASSIFICATION OF THE APPLICATION (Int. Cl.4) |
|---|---|---|---|
| Y | EP-A-0 011 190 (BAYER)<br><br>* Page 4, line 27 - page 5, line 5; example 7 *<br><br>----- | 1,4-6,<br>9,10 | |
| | | | TECHNICAL FIELDS SEARCHED (Int. Cl.4) |
| | The present search report has been drawn up for all claims | | |

| Place of search<br>THE HAGUE | Date of completion of the search<br>12-12-1984 | Examiner<br>BERTE M.J. |

EPO Form 1503 03 82